# EUROPEAN PATENT APPLICATION

(11) **EP 2 998 000 A1**
(43) Date of publication of application: **23.03.2016**
(21) Application number: 15185432.0
(22) Date of filing: 16.09.2015
(51) Int. Cl.: A61P 35/00, A61K 35/74, G01N 33/50, A61K 45/06

(54) **COMBINATION THERAPY FOR CANCER**

(30) Priority: 16.09.2014 EP 14185033
(71) Applicant: University College Cork-National University of Ireland, Cork, Cork (IE)
(72) Inventor: TANGNEY, Mark, Co. Cork (IE); LEHOURITIS, Panos, 1146 Athens (GR)
(74) Representative: Purdy, Hugh Barry

(57) **Abstract**

A combination comprising an effective dose of a chemotherapeutic-activating wild-type bacterium and an effective dose of a chemotherapeutic agent that is responsive to the chemotherapeutic activating wild-type bacterium, **for use in** a method of preventing or treating cancer in a mammal.

## Description

### Introduction

Tumour responses to chemotherapy vary, and deeper insight into the reasons for therapeutic failure with certain tumours while other apparently similar tumours respond well, stands to guide and improve existing therapeutic regimes, while informing the development of new treatments. Bacteria have been linked with various cancers in a number of ways. For example, bacterial-induced inflammation has been linked with cancer promotion and progression, via indirect distal effects from the GIT microbiome, or directly such as in the case of *Helicobacter pylori.* Recent research in experimental tumours revealed that gut bacteria may influence the outcome of chemotherapy indirectly via influencing the immune system. For decades, naturally occurring bacteria of different types have been isolated from patient tumours of various histological types. In parallel to this, it is well known that deliberate systemic administration of bacteria to animals or patients results in selective replication within solid tumours. The reasons for tumour tissue targeting by bacteria and their proliferation within them believed to be multifactorial, with a number of key features distinguishing the tumour phenotype from normal tissues: 'Leaky' tumour vasculature may provide bacterial access to tissue; the immune-suppressed environment of the tumour protects bacteria from immune surveillance; the low oxygen potential of necrotic and hypoxic regions of tumours facilitates anaerobic bacterial growth (unlike other tissues); tumour necrosis provides nutrients favourable to bacterial growth. The bacterial populations naturally present within malignant and non-malignant tissue of the breast, and the presence of a range of bacteria in the breast tissue of cancer patients, has recently been reported.

### Statements of Invention

The present invention is based on the finding that certain wild-type bacteria can have an effect on certain chemotherapy drugs. The effect can be positive, where the cytotoxic effect of the drug on a tumour is enhanced in the presence of a specific wild-type bacteria, or negative, where the cytotoxic effect is inhibited in the presence of the specific wild-type bacteria. Table 1 shows the effect of a two wild-type bacteria (*E. coli and Listeria welshimeri*) on the cytotoxic effect of 29 conventional chemotherapeutic drugs in vitro, in which 5 have improved cytotoxic effect, 10 have a reduced cytotoxic effect, and 14 are unaffected, relative to a reference cytotoxic effect.

The invention provides novel tailored therapies for cancer in individuals that is informed by (i) a synergistic combination of a specific wild-type bacterium and a chemotherapeutic drug that responds to the specific wild-type bacterium, or (ii) a combination of a chemotherapeutic drug and an anti-bacterial agent, in which the anti-bacterial agent is directed against a wild-type bacterium known to inhibit the chemotherapeutic effect of the chemotherapeutic drug.

In a first aspect, the invention provides a method for identifying a chemotherapeutic agent that is a responsive to a specific wild-type bacterium (hereafter "wild-type bacterium-responsive chemotherapeutic agent") comprising the steps of incubating the specific wild-type bacterium with a candidate chemotherapeutic agent, separating the candidate chemotherapeutic agent from the wild-type bacterium, incubating the candidate chemotherapeutic agent with a cancer cell, and determining whether there is an increase in a cytotoxic effect of the candidate chemotherapeutic agent on the cancer cell relative to a reference cytotoxic effect, wherein an increase in cytotoxic effect relative to the reference cytotoxic effect indicates that the candidate chemotherapeutic agent is a wild-type bacterium responsive chemotherapeutic agent.

Thus, for any specific wild-type bacterium, it is possible to identify chemotherapeutic agents that are responsive to the specific wild-type bacterium. It is also possible to identify wild-type bacteria that activate specific chemotherapeutic agents (hereafter "chemotherapeutic activating wild-type bacteria" or "chemotherapeutic activating wild-type bacterium").

The invention also provides a method for treating cancer in a mammal comprising administering to the mammal a synergistic combination comprising an effective dose of a chemotherapeutic-activating wild-type bacterium and an effective dose of a chemotherapeutic agent that is responsive to the chemotherapeutic activating wild-type bacterium (hereafter "wild-type bacterium-responsive chemotherapeutic agent").

The invention also provides a pharmaceutical composition comprising a chemotherapeutic activating wild-type bacterium, a wild-type bacterium-responsive chemotherapeutic agent, and a pharmaceutically acceptable excipient.

The invention also provides a chemotherapeutic-activating wild-type bacterium in combination with a chemotherapeutic agent that is responsive to the chemotherapeutic activating wild-type bacterium, for use in the prevention or treatment of cancer.

The invention also relates to a method of treating or preventing a cancer in an individual comprising the steps of assay a tumour sample from the individual for the presence of a wild-type bacterium, wherein when the presence of a wild-type bacterium is detected, administering to the individual a chemotherapeutic agent that is responsive to the chemotherapeutic activating wild-type bacterium

In another aspect, the invention provides a method for identifying a chemotherapeutic agent that is non-responsive to specific wild-type bacterium (hereafter "wild-type bacterium non-responsive chemotherapeutic agent") comprising the steps of incubating the specific wild-type bacterium with a candidate chemotherapeutic agent for a period of time, separating the candidate chemotherapeutic agent from the wild-type bacterium, incubating the candidate chemotherapeutic agent with a cancer cell, and determining whether there is an decrease in a cytotoxic effect of the candidate chemotherapeutic agent on the cancer cell relative to a reference cytotoxic effect, wherein a decrease in cytotoxic effect relative to the reference cytotoxic effect indicates that the candidate chemotherapeutic agent is a wild-type bacterium non-responsive chemotherapeutic agent.

Thus, for any specific wild-type bacterium, it is possible to identify chemotherapeutic agents that are non-responsive to (i.e. inhibited by) the specific wild-type bacterium, and therefore formulate or prescribe therapies accordingly, for example providing a cancer therapy comprising a wild-type bacterium non-responsive chemotherapeutic agent and an anti-bacterial agent effective against the wild-type bacterium.

The invention therefore also provides for a method for preventing or treating cancer in a mammal comprising administering to the mammal a combination therapy comprising an effective dose of a wild-type bacterium non-responsive chemotherapeutic agent and an effective dose of an anti-bacterial agent effective against the wild-type bacterium. The anti-bacterial agent may be specific to the wild-type bacterium, or a broad-spectrum anti-bacterial agent.

The invention also provides a pharmaceutical composition comprising a wild-type bacterium non-responsive chemotherapeutic agent, an anti-bacterial agent effective against the wild-type bacterium, and a pharmaceutically acceptable excipient.

The invention also provides for a wild-type bacterium non-responsive chemotherapeutic agent and an anti-bacterial agent effective against the wild-type bacterium, for use in the prevention or treatment of cancer characterised by the presence of the wild-type bacterium.

The invention also relates to a method of identifying a suitable chemotherapeutic agent for a mammal having a tumour comprising the steps of assaying the tumour for the presence of at least one chemotherapeutic activating wild-type bacterium, wherein when the presence in the tumour of the at least one chemotherapeutic activating wild-type bacterium is confirmed, identifying a wild-type bacterium-responsive chemotherapeutic agent as a suitable chemotherapeutic agent for the mammal.

The invention also relates to a method for the prevention or treatment of cancer in an individual comprising the steps of identifying a suitable chemotherapeutic agent for the mammal according to a method of the invention, and then administering an effective dose of the identified chemotherapeutic agent to the mammal.

The invention also relates to a method of treating or preventing a cancer in an individual comprising the steps of assay a tumour sample from the individual for the presence of a chemotherapeutic inhibiting wild-type bacteria, wherein when the presence of chemotherapeutic-inhibiting wild-type bacteria is detected, administering to the individual an anti-bacterial agent directed against the chemotherapeutic-inhibiting wild-type bacteria and a wild-type bacteria non-responsive chemotherapeutic agent.

The invention also relates to a method of biotransforming a chemotherapeutic agent comprising the step of incubating the chemotherapeutic agent with a chemotherapeutic-activating wild-type bacteria, or an extract of the chemotherapeutic-activating wild-type bacteria (for example, a cell lysis extract), for a period of time sufficient to allow the chemotherapeutic-activating wild-type bacteria or extract thereof convert the chemotherapeutic agent to a biotransformed chemotherapeutic agent, and then optionally separating the biotransformed chemotherapeutic agent from the bacteria or extract thereof..

### Brief Description of the Figures

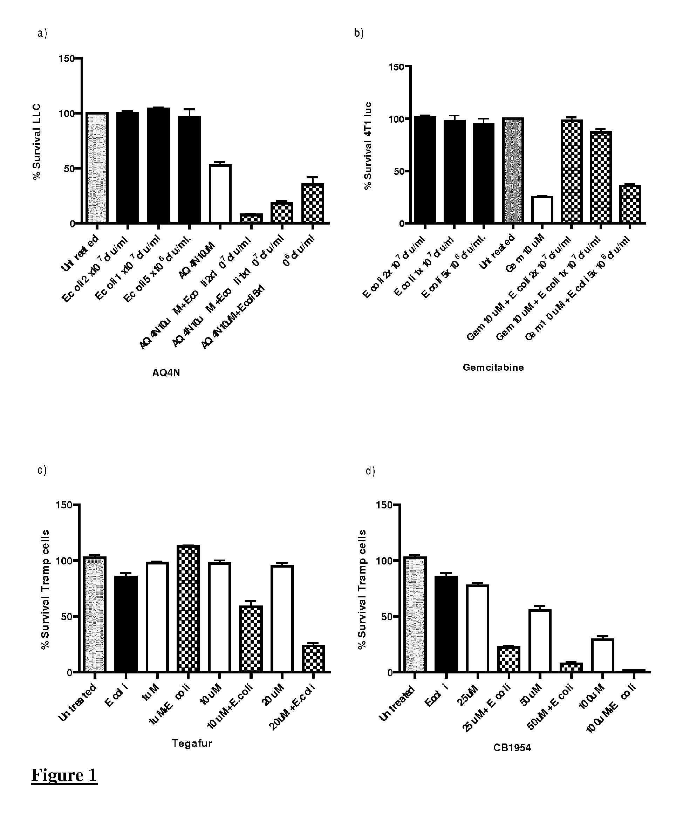

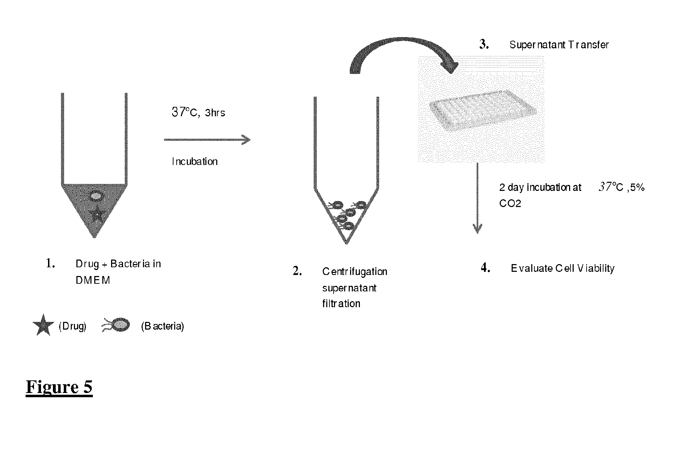

**Figure 1****:** *Tumour Cell Survival*
   Cell survival assay. See Figure 5 for assay schematic. **a)** *E.coli* at different cfu/ml were co-incubated with AQ4N (10 µM) after which the supernatant was applied directly to LLC cells (P < 0.01). **b)** *E. coli* at different cfu/ml were co-incubated with gemcitabine (10 µM) after which the supernatant was directly applied to 4T1 Luc cells (P < 0.01). c) *E.coli* was co-incubated with Tegafur at the indicated concentrations after which the supernatant was directly applied to TRAMP cells (P < 0.01). Data represent the average and standard error of three technical replicates.

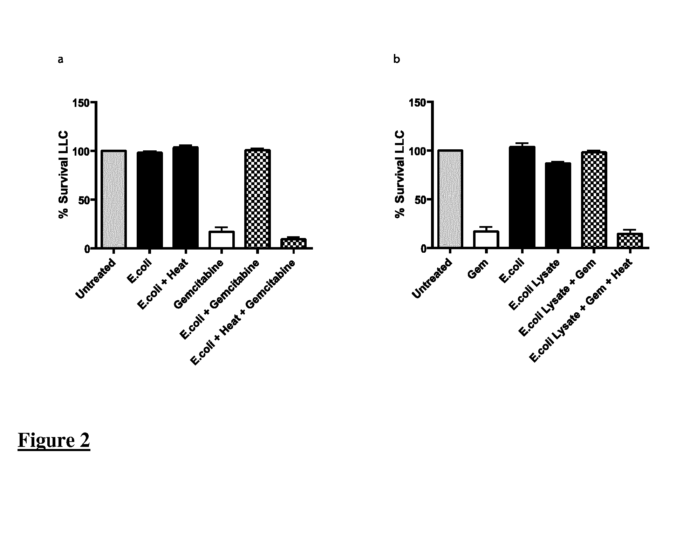

**Figure 2****:** *Cell lysate assays*
   **a)** Tumour cell survival assay stained with MTS. Gemcitabine (10 µM) was incubated with live or heat killed *E. coli* (P < 0.001). **b)** Tumour cell survival assay. Gemcitabine (10 µM) was incubated with either bacterial lysate (equivalent amounts to cell survival assay live bacteria dosages) alone or bacterial lysate that has been heat inactivated (P < 0.001). Data represent the average and standard error of three technical replicates.

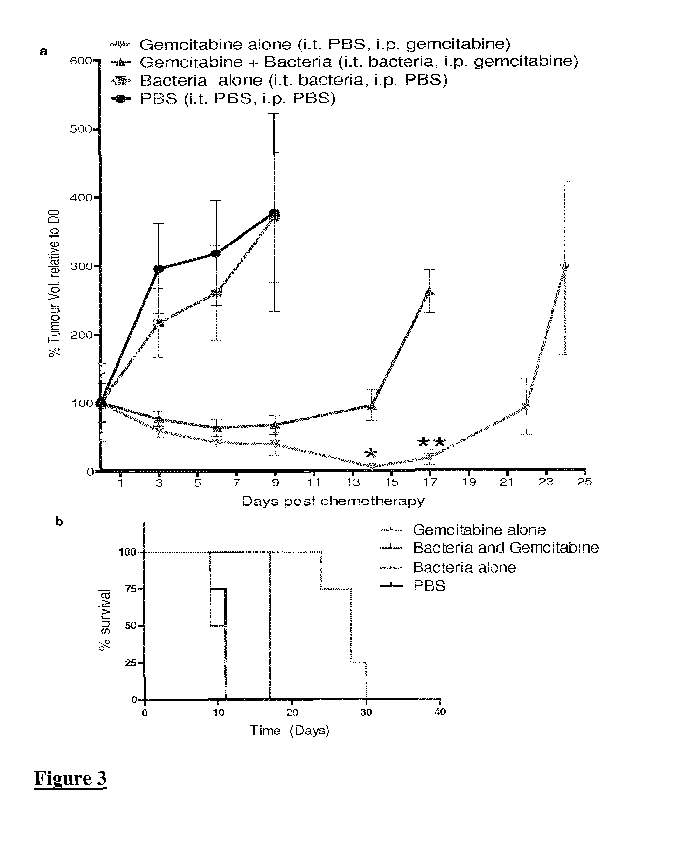

**Figure 3****:** *E. coli decreases the efficacy of gemcitabine in vivo*
   Subcutaneous flank CT26 tumours growing in Balb/c mice were injected i.t with bacteria or PBS vehicle alone. Gemcitabine (60 mg/kg) was injected i.p. five times at three day intervals. **a)** Tumour volume (%) relative to the first day of gemcitabine injection (day 0) is shown. *P < 0.03, **P = 0.002 for gemcitabine alone versus gemcitabine + bacteria. **b)** The median survival post Day 0 of the gemcitabine + bacteria group was significantly less than that of the gemcitabine alone group (17 days vs. 28 days +/- 1.25; P = 0.008). Data are expressed as mean ± SEM of 4 to 8 individual mice per group.

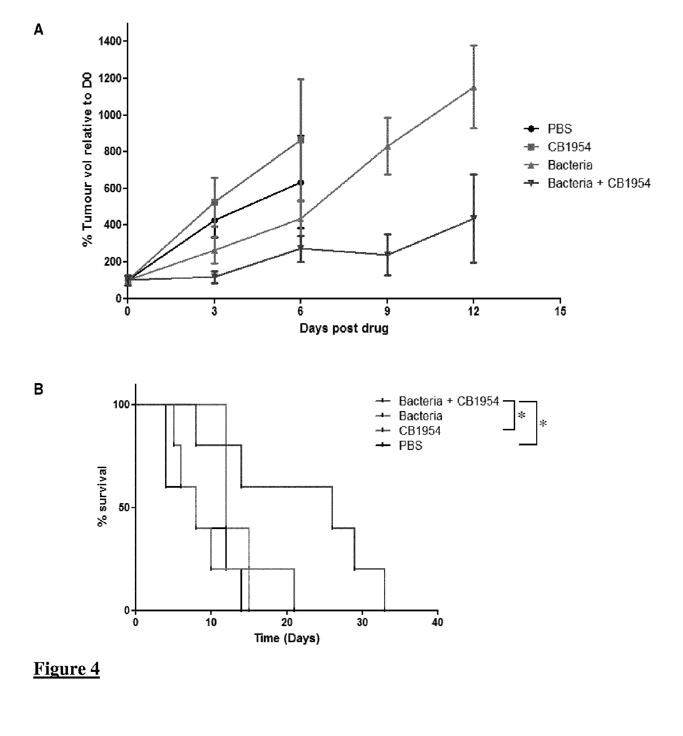

**Figure 4****:** *E. coli increases the cytotoxicity of CB1954*
   Subcutaneous flank CT26 tumours growing in Balb/c mice were injected i.t with bacteria or PBS vehicle alone. CB1954 (20 mg/kg) was injected i.p. for the duration of the experiment at 3 day intervals. a) Tumour volume (%) relative to the first day of CB1954 injection (day 0) is shown. b) The median survival post Day 0 of the CB1954 + bacteria group was significantly greater than that of the CB1954 alone group (26 days vs. 8 days. P = 0.0374). Data are expressed as mean ± SEM of 3-5 individual mice per group.
**Figure 5****:** *Tumour survival assay*
   **1.** Bacteria and Drug are mixed in DMEM medium, and co-incubated for 3 h at 37°C. **2.** The drug is separated from bacteria using centrifugation and filtration. **3.** The filter sterilised DMEM containing the transformed drug is applied to tumour cells in a 96 well plate. **4.** Tumour cell viability is evaluated via the MTS assay for end point measurements.

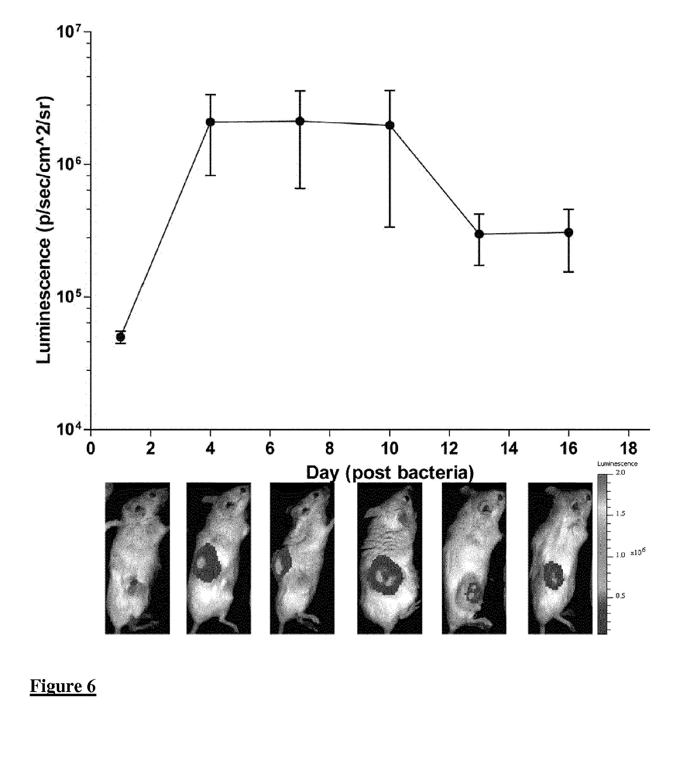

**Figure 6****:** *Growth of i.t. administered bacteria in mouse tumours*
   1x10⁶ lux-expressing *E. coli* was i.t. administered to s.c. CT26 flank tumours growing in Balb/c mice. Viable, luminescent bacteria were detected at various time points specifically in the tumour using whole body imaging. A graphical representation of tumour luminescence over time plotted as the mean +/- SEM for Four mice, with a representative mouse image under corresponding time points.

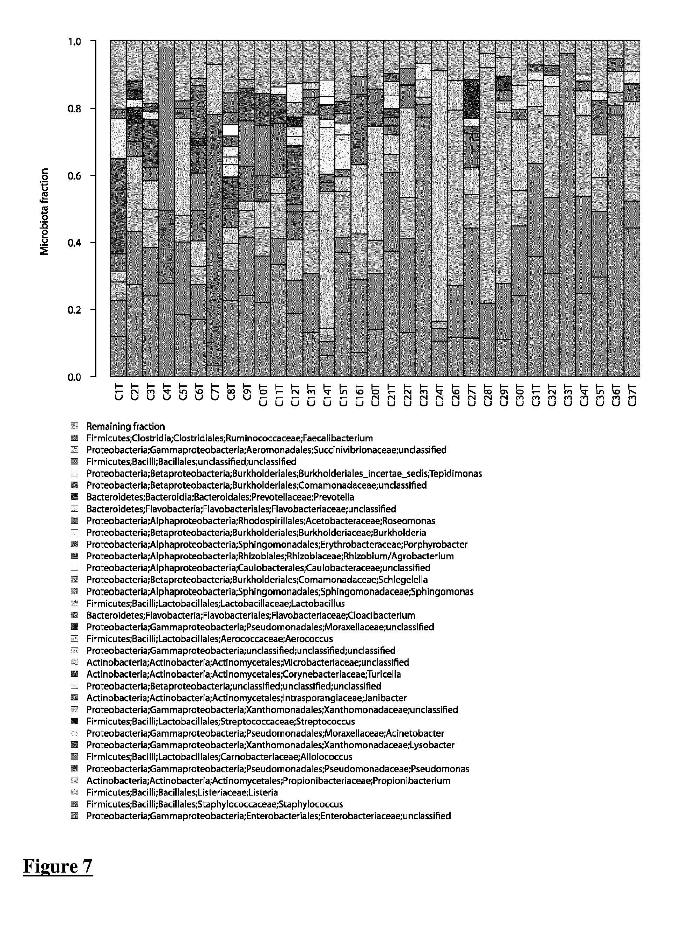

**Figure 7****:** Analysis of tumour microbiota

### Detailed Description of the Invention

### Definitions:

"Cancer": means the group of diseases involving abnormal cell growth with the potential to spread to distant or local sites, especially means a malignant tumour. Typically, the cancer is selected from the group comprising: esophagogastric cancer; fibrosarcoma; myxosarcoma; liposarcoma; chondrosarcoma; osteogenic sarcoma; chordoma; angiosarcoma; endotheliosarcoma; lymphangiosarcoma; lymphangioendotheliosarcoma; synovioma; mesothelioma; Ewing's tumor; leiomyosarcoma; rhabdomyosarcoma; colon carcinoma; pancreatic cancer; breast cancer; ovarian cancer; prostate cancer; squamous cell carcinoma; basal cell carcinoma; adenocarcinoma; sweat gland carcinoma; sebaceous gland carcinoma; papillary carcinoma; papillary adenocarcinomas; cystadenocarcinoma; medullary carcinoma; bronchogenic carcinoma; renal cell carcinoma; hepatoma; bile duct carcinoma; choriocarcinoma; seminoma; embryonal carcinoma; Wilms' tumor; cervical cancer; uterine cancer; testicular tumor; lung carcinoma; small cell lung carcinoma; bladder carcinoma; epithelial carcinoma; glioma; astrocytoma; medulloblastoma; craniopharyngioma; ependymoma; pinealoma; hemangioblastoma; acoustic neuroma; oligodendroglioma; meningioma; melanoma; retinoblastoma; and leukemias.

"Treatment": means a course of action / dosing regime that either inhibits, delays or prevents the progression of cancer, including cancer metastasis, or that inhibits, delays or prevents the recurrence of cancer, including cancer metastasis, or that prevents or hinders the onset or development of cancer in an individual.

"Prevention": means prevention of the occurrence or recurrence of cancer, at a local or distant site, typically following the withdrawal of chemotherapeutic drugs in an individual diagnosed with cancer.

"Chemotherapeutic agent": means an agent that induces cancerous cells to commit to cell death. Suitable chemotherapeutic agents will be known to those skilled in the art. Such chemotherapeutic agents include but are not limited to; alkylating agents, anti-metabolites, plant alkyloids and terpenoids, topoisomerase inhibitors, anti-tumour antibiotics and platinum based compound including but not limited to cisplatin, carboplatin and oxaliplatin. Examples of suitable chemotherapeutic anti-metabolites include, purine analogues not limited to azathoprine, mercaptopurine, tioguanine and fludarabine; pyrimidine analogues not limited to 5-fluorouracil (5-FU), floxuridine and cytosine arabinoside; antifolates not limited to methotrexate, trimethoprim, pyrimethamine and pemetrexed. Suitably, the chemotherapeutic agent is an anti-metabolite.

"Wild-type bacteria" or "wild-type bacterium": means naturally occurring bacteria that are not genetically modified. Preferably, the term means wild-type bacteria known to inhabit mammalian tumours, especially malignant mammalian tumours, (hereafter "mammalian tumor resident wild-type bacterium") examples of which are described in the literature and Fig. 7. In one embodiment of the invention the wild-type bacteria is a species of the taxa/genera *E.coli,* typically *E.coli nissle.* In another embodiment the wild-type bacteria is a species of the taxa/genera *Listeria,* typically *Listeria welshimeri*. In another embodiment the wild-type bacteria is a species of the taxa/genera *Enterobacteriacae.* In another embodiment the wild-type bacteria is a species of the taxa/genera *Staphylococcus.* In another embodiment the wild-type bacteria is a species of the taxa/genera *Propionibacterium.* In another embodiment the wild-type bacteria is a species of the taxa/genera *Pseudomonas.* In another embodiment the wild-type bacteria is a species of the taxa/genera *Alloiococcus.* In another embodiment the wild-type bacteria is a species of the taxa/genera *lysobacter.* In another embodiment the wild-type bacteria is a species of the taxa/genera *Acinetobacter.* In another embodiment the wild-type bacteria is a species of the taxa/genera *Streptococcus.* In another embodiment the wild-type bacteria is a species of the taxa/genera *Xanthomonadaceae.* In another embodiment the wild-type bacteria is a species of the taxa/genera *Janibacter.* In another embodiment the wild-type bacteria is a species of the taxa/genera *Betaproteobacteria.* In another embodiment the wild-type bacteria is a species of the taxa/genera *Tarricella.* In another embodiment the wild-type bacteria is a species of the taxa/genera *Microbacteriaceae.* In another embodiment the wild-type bacteria is a species of the taxa/genera *Gammaproteobacteria.* In another embodiment the wild-type bacteria is a species of the taxa/genera *Aerococcus.* In another embodiment the wild-type bacteria is a species of the taxa/genera *Moraxellaceae.* In another embodiment the wild-type bacteria is a species of the taxa/genera *Cloacibacterium.* In another embodiment the wild-type bacteria is a species of the taxa/genera *Lactobacillus.* In another embodiment the wild-type bacteria is a species of the taxa/genera *Sphingomonas.* In another embodiment the wild-type bacteria is a species of the taxa/genera *Scxhlegella.* In another embodiment the wild-type bacteria is a species of the taxa/genera *Caulobacteraceae*. In another embodiment the wild-type bacteria is a species of the taxa/genera *Rhizobiiaceae*. In another embodiment the wild-type bacteria is a species of the taxa/genera *Porphyrobacter.* In another embodiment the wild-type bacteria is a species of the taxa/genera *Burkholderia.* In another embodiment the wild-type bacteria is a species of the taxa/genera *Roseomonas.* In another embodiment the wild-type bacteria is a species of the taxa/genera *Flavobacteriaceae.* In another embodiment the wild-type bacteria is a species of the taxa/genera *Prevotella.* In another embodiment the wild-type bacteria is a species of the taxa/genera *Comamonadacae.* In another embodiment the wild-type bacteria is a species of the taxa/genera *Tepidomonas.* In another embodiment the wild-type bacteria is a species of the taxa/genera *Bacillales.* In another embodiment the wild-type bacteria is a species of the taxa/genera *Succinivivrionaceae*. In another embodiment the wild-type bacteria is a species of the taxa/genera *Faecalibacterium.* Enterobacteriacae, Staphylococcus, Listeria, Propionibacterium, Pseudomonas, Alloiococcus, lysobacter, Acinetobacter, Streptococcus, Xanthomonadaceae, Janibacter, Betaproteobacteria, Turicella, Microbacteriaceae, Gammaproteobacteria, Aerococcus, moraxellaceae, Cloacibacterium, Lactobacillus, Sphingomonas, Schlegella, caulobacteraceae, Rhizobiiaceae, Porphyrobacter, Burkholderia, Roseomonas, Flavobacteriaceae, Prevotella, Comamonadaceae, Tepidimonas, Bacillales, Succinivibrionaceae, Faecalibacterium.

"Cytotoxic effect": means the inhibition of cell growth or induction of cell death.

"Reference cytotoxic effect": means the cytotoxic effect of a chemotherapeutic agent against a cancer cell carried out in the absence of a wild-type bacteria, or where the chemotherapeutic agent is not pre-incubated with the wild-type bacteria.

"Synergistic combination": means a combination of actives that is used in cancer therapy and comprises a wild-type bacteria known to activate (i.e. increase the cytotoxic effect) of a specific chemotherapeutic agent and a chemotherapeutic agent known to be activated by the wild-type bacteria. The actives in the synergistic combination may be administered together (co-administration) or separately, for example at different times of the day or on different days. Preferably, the bacteria is administered to the patient prior to the drug, typically at least 1, 3, 5, 7, 9, 11, 13, 15, 17 or 19 days prior to administration of the chemotherapeutic agent. The synergistic combination may be provided in a form for co-administration of the actives (i.e. in the form of a unit dose product containing both actives) or for sequential or separate administration of the actives (i.e. a kit comprising the two actives provided separately, for example two unit dose products).

"Wild-type bacteria-responsive chemotherapeutic agent" or "chemotherapeutic agent that is responsive to the chemotherapeutic activating wild-type bacteria": means a chemotherapeutic agent that is activated by the wild-type bacteria (i.e. exhibits an increased cytotoxic effect or is converted to a more cytotoxic form in the presence of the bacteria). Without being bound by theory, it is believed that the chemotherapeutic agent is biotransformed by one or more specific wild-type bacteria to a more cytotoxic chemotherapeutic form. Examples of wild-type bacteria-responsive chemotherapeutic agent are provided below and include Tegafur, Fludarabine de phosphate, 5-fluorocysteine, 6-mercaptopurine-2'-deoxyriboside, and AQ4N which are responsive to *E.coli, L. welshimeri*, or both.

"Chemotherapeutic-activating wild type bacterium": means wild-type bacterium that are capable of increasing the cytotoxic effect of one or more chemotherapeutic agents. Examples of chemotherapeutic-activating wild type bacteria include *E.coli* and *L. welshimeri*.

"Chemotherapeutic-inhibiting wild type bacterium": means wild-type bacterium that are capable of decreasing the cytotoxic effect of one or more chemotherapeutic agents. Examples of chemotherapeutic-inhibiting wild type bacteria include *E.coli* and *L. welshimeri*.

"Effective dose": as applied to a wild-type bacterium means a dosage of the bacteria that is sufficient to increase the cytotoxic effect of the wild-type bacteria responsive chemotherapeutic agent *in vivo.* In one embodiment, an effective dose is at least 1 x 10⁷, 1 x 10⁸ or 1 x 10⁹ bacteria per millilitre in a suitable carrier.

"Biotransformed chemotherapeutic agent": means a chemotherapeutic agent that is biotransformed by a chemotherapeutic-activating wild type bacterium to more cytotoxic form.

"Effective dose": as applied to a chemotherapeutic agent means an amount of a chemotherapeutic agent which results in partial or total inhibition in the progression of cancer and/or prevents or inhibits the recurrence of cancer following withdrawal from an anti-cancer regime. In a particular, a therapeutically effective amount of a chemotherapeutic agent should be taken to mean an amount that results in a clinically significant number of cancer cells being killed. An effective amount can be readily determined by the attending diagnostician, as one skilled in the art, by the use of known techniques and by observing results obtained under analogous circumstances. In determining the effective amount or dose of compound administered, a number of factors are considered by the attending diagnostician, including, but not limited to: the type of chemotherapeutic agent; species of mammal; its size, age, and general health; the specific disease involved; the degree of or involvement or the severity of the disease; the response of the individual patient; the particular compound administered; the mode of administration; the bioavailabilty characteristics of the preparation administered; the dose regimen selected; the use of concomitant medication; and other relevant circumstances. As an example, the following doses may be employed:
Cisplatin: high dose = 6.9mg/kg; low dose = 2mg/kg
Lithium Chloride: high dose = 14.5/17mg/kg; low dose = 4.5/10mg/kg
Rapamycin: high dose = 2mg/kg; low dose = 0.6mg/kg
5-Fluorouracil: high dose = 87mg/kg; low dose = 8/12mg/kg

In this specification, the term "administering" should be taken to include any form of delivery that is capable of delivering the chemotherapeutic agent and the wild-type bacteria (or anti-bacterial agent) to cancer cells including intravenous delivery, oral delivery, intramuscular delivery, intrathecal delivery, transdermal delivery, inhaled delivery and topical delivery. Methods for achieving these means of delivery will be well known to those skilled in the art of drug delivery. The term should also encompass co-administration of the two actives, or administration at separate times. For example, the actives may be administered on alternate days, or on the same day at different times, or on different days of the week. Preferably, the bacteria is administered to the patient prior to the drug, typically at least 1, 3, 5, 7, 9, 11, 13, 15, 17 or 19 days prior to administration of the chemotherapeutic agent.

### Experimental

### Materials and Methods:

### Bacteria and cell lines

*E. coli* Nissle 1917 (UCC culture collection) was cultivated aerobically in L-Broth or L-Agar (Sigma) at 37 °C. Bioluminescent *E. coli* was described by us previously (1) and cultured in the presence of 300 mg/ml erythromycin. *Listeria welshimeri* Serovar 6B SLCC5334 was purchased from ECACC and cultivated at 37 °C in Brain Heart Infusion (BHI) medium. Lewis Lung Carcinoma (LLC), 4T1 (mouse mammary carcinoma) and CT26 (mouse colorectal carcinoma) cells were purchased from ATCC and were propagated according to the supplier's instructions. The murine recycled prostate cancer cell line TRAMPC1 was kindly provided by Ciavarra RP (2) of Eastern Virginia Medical School, Norfolk USA, and propagated as described in (3).

### Drugs

All drugs were purchased from Sigma except: Etoposide Phosphate (Santa Cruz), Capecitabine (Santa Cruz), AQ4N (R&D), Nelarabine (A&B), and Vidarabine (Santa Cruz). Drugs were resuspended in H₂O or DMSO, with appropriate control vehicle utilised accordingly in all experiments.

### Cell cytotoxicity assay

Microtitre plates (96-well) were pre-seeded with 4000 cells/well in appropriate medium for each cell line and allowed to attach overnight. On the day of the assay, bacteria were cultured to log-phase and a subculture ratio, corresponding to an OD₆₀₀ₙₘ of 0.2 determined for each strain, was exposed for 2 h (4 h in the case of IC50s) to drug in falcon tubes containing DMEM in a tissue culture incubator. Falcon content was filter-sterilized to remove presence of bacteria (using 0.2 µm pore filters (Starsted) before adding 200 µl per well. Plates were incubated until cells in control (untreated) had achieved confluent growth. Cytotoxicity was quantified using an MTS staining with the Cell Titre 96 AQueous One solution Cell Proliferation Assay (Promega). The plates were incubated with normal media (80 µl) and MTS solution (20 µl) in a 37 °C incubator for approximately 2 h (until distinct change of colour occurred).

### Bacterial lysis and heat inactivation

Bacteria were heat inactivated at 95 °C for 40 min. Lysis was facilitated by sonication using three 10 sec pulses (at 20 khtz, 50 W). Between each pulse, samples were incubated on ice for 30 seconds. A 20-fold drop in optical density was considered sufficient lysis.

### Murine experiments

*Animals and Tumour Induction:* Mice were kept at a constant room temperature (22 °C) with a natural day/night light cycle in a conventional animal colony. Standard laboratory food and water were provided *ad libitum.* Before experiments, the mice were afforded an adaptation period of at least 7 days. Female mice in good condition, without fungal or other infections, weighing 16-22 g and of 6-8 weeks of age, were included in experiments (Harlan, Oxfordshire, UK). For CT26 tumour induction, 6 X 10⁵ cells suspended in 200 µl of serum-free culture medium were injected subcutaneously (s.c.) into the flank. The viability of cells used for inoculation was greater than 95% as determined by visual count using a haemocytometer and Trypan Blue Dye Exclusion (Gibco), or the Nucleocounter system (ChemoMetec, Bioimages Ltd, Cavan, Ireland). Following tumour establishment, tumours were allowed to grow and develop and were monitored three times weekly. Tumour volume was calculated according to the formula *V* = (*ab*²) Π/6, where *a* is the longest diameter of the tumour and *b* is the longest diameter perpendicular to diameter *a.* When tumours reached approximately 100 mm³ in volume, mice were randomly divided into experimental groups.

*Bacterial and drug administration:* Overnight cultures of *E. coli* were re-inoculated into fresh LB (1/50 dilution) and incubated shaking at 37 °C until they reached an OD₆₀₀ of 0.7. Cells were then washed twice in PBS. Tumours were administered 10⁶ *E. coli* in an injection volume of 50 µl by intratumoural (i.t.) injection. The viable count of each inoculum was determined by retrospective plating onto LB agar. Two hours post bacterial administration, drug (gemcitabine 60 mg/kg or CB1954 20 mg/kg) was administered by intra-peritoneal (i.p.) injection in an injection volume of 50 µl. The drug was subsequently administered on days 3, 6, 9, and 11 and animals not receiving the drug were administered an equal volume of PBS vehicle.

### Statistical analysis

For bioinformatics, statistical analysis was performed in R. For biological *in vitro* and *in vivo* assays, two-sided, paired student's t test with 95% confidence or Mann Whitney U test were employed to investigate statistical differences, using GraphPad Prism or Microsoft Excel 12. Statistical significance of survival between groups in murine experiments was determined using the Log-rank (Mantel-Cox) Test.

### Results:

### Effects of bacteria on chemotherapeutic drug cytotoxicity in vitro

Various bacteria and cancer cell lines were examined *in vitro* for changes in the efficacy of cancer cell killing mediated by a range of chemotherapeutic agents. An *in vitro* assay was developed to facilitate drug and bacteria screening as described in experimental procedures and Figure 5. Briefly, individual bacterial strains and drugs were co-incubated for 3 h, before separation via centrifugation and filtration, after which the supernatant containing the metabolized drug plus any derivatives was applied directly to growing tumour cells. Cell growth was visually monitored microscopically over time, and cell viability measured after 2 days using the classical MTS assay for end point measurements. Assay validation proceeded with several drugs whose effects were observed to be altered in the presence of *E. coli.* A drug whose efficacy was enhanced (Figure 1a-AQ4N), and a drug whose efficacy was decreased (Figure 1b-gemcitabine) by *E. coli* were examined with a range of bacterial concentrations. In both cases, a dose response (P < 0.01) was achieved using three different bacterial concentrations, indicating that changes in drug cytotoxicity were originating from the bacteria. A dose response using different drug concentrations (P < 0.01) was also demonstrated with the drug tegafur (Figure 1c).

Having identified the most frequently occurring bacterial species in patient tumours, studies proceeded with bacterial strains representing two high-occurrence species; Gram-negative non-pathogenic *E. coli* and Gram-positive *Listeria welshimeri* (also non-pathogenic). Twenty nine agents from a number of different drug classes (mechanisms of action) were examined, the majority of which represent commonly-employed FDA-licenced drugs. The results are summarised in Table 1. Of 29 drugs examined, the cancer killing efficacies of 10 were found to be decreased, while in general the same bacteria increased the efficacies of 5 others. 14 drugs did not display any difference in efficacy following bacteria co-incubation. Observed effects on drug cytotoxicity were not identical between *E. coli* and *L. welshimeri* (where tested), with *E. coli* increasing the effect of Tegafur (unlike *L. welshimeri*), and decreasing the effect of Vidarabine, Gemcitabine and Etoposide phosphate (unlike *L. welshimeri*).

### Mechanism of action of bacteria on drugs

In order to investigate if the observed effects were mediated by biochemical modification of the drugs, rather than any physical bacterial titration of the drug (in the case of drugs with decreased activity), the *in vitro* assay was performed with bacteria treated in a number of ways prior to incubation with drug. Gemcitabine (cytotoxicity decreased by bacteria) was examined with supernatant from *E. coli* which had been lysed by sonication. Similar decreases in cytotoxicity were observed when gemcitabine was incubated with this lysate as with intact bacteria (Figure 2). Upon heat-inactivation of the lysate (or intact bacteria) at 95 °C for 20 min, no reduction in cytotoxicity was evident (P < 0.001), suggesting the involvement of enzymatic activity.

### Murine models of intratumoural bacterial effects on chemotherapy

The ability of *E. coli* and other bacterial species to selectively replicate in tumours over time in various murine models has been described previously (4, 5). In this study, the growth of *E. coli* in CT26 tumours was initially validated following intratumoural (i.t.) administration (Figure 6). The chemoresistance observed *in vitro* with a sample drug (gemicitabine) was examined in this model. Mice bearing CT26 tumours were i.t. injected with *E. coli* or PBS, and intraperitoneally (i.p.) injected with gemcitabine or PBS and monitored over time (Figure 3). The PBS:PBS and Bacteria:PBS groups showed the greatest tumour volumes over time. Significantly increased tumour volume was observed in the gemcitabine + bacteria group compared with the gemcitabine alone group at various time points (P < 0.03; Figure 3a). Survival was significantly reduced in the gemcitabine + bacteria group compared with the gemcitabine alone group (17 days vs. 28 days +/- 1.25; P = 0.004; Figure 3b). These data indicate reduced gemcitabine anti-tumour activity in tumours containing bacteria. Bacteria alone did not significantly affect tumour volume relative to PBS administration (P > 0.2), although some reduction is evident in bacteria administered tumours, suggesting that the reduced gemcitabine effect may be partially masked.

The ability of the same bacterial species to upregulate the cytotoxicity of another drug, CB 1954, was also examined in this model. A significant increase in median survival (26 days vs. 8 days. P = 0.028) was observed in the CB1954 + bacteria group compared with the CB1954 alone group (Figure 4), indicating drug activation by the intratumoural bacteria.

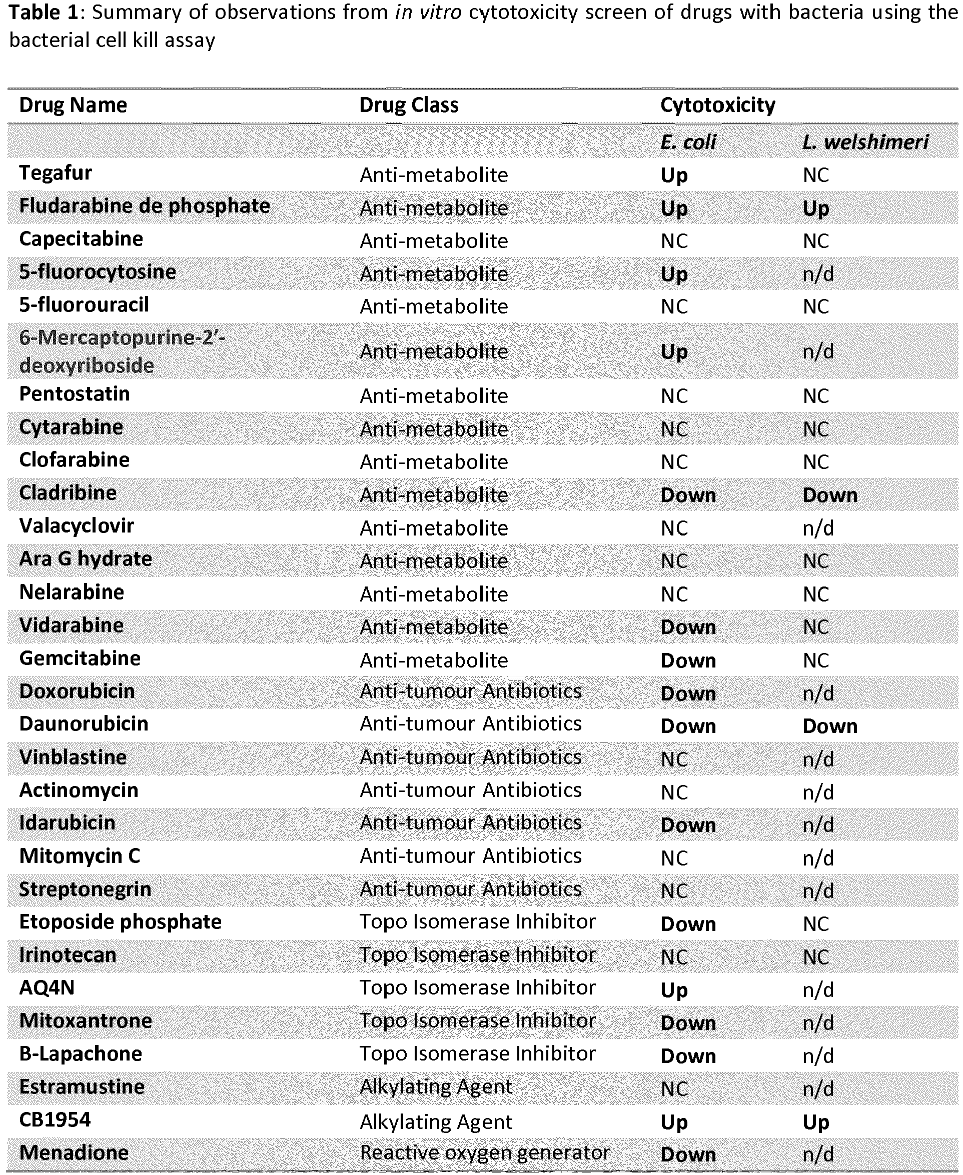

### References

1. Cronin M, Akin AR, Collins SA, Meganck J, Kim JB, Baban CK, et al. High resolution in vivo bioluminescent imaging for the study of bacterial tumour targeting. PloS one. 2012;7:e30940.
2. Somers KD, Brown RR, Holterman DA, Yousefieh N, Glass WF, Wright GL, Jr., et al. Orthotopic treatment model of prostate cancer and metastasis in the immunocompetent mouse: efficacy of flt3 ligand immunotherapy. Int J Cancer. 2003;107:773-80.
3. Ahmad S, Casey G, Sweeney P, Tangney M, O'Sullivan GC. Prostate stem cell antigen DNA vaccination breaks tolerance to self-antigen and inhibits prostate cancer growth. Mol Ther. 2009;17:1101-8.
4. Cronin M, Akin AR, Collins SA, Meganck J, Kim JB, Baban CK, et al. High resolution in vivo bioluminescent imaging for the study of bacterial tumour targeting. PloS one. 2012;7:e30940.
5. Cronin M, Morrissey D, Rajendran S, El Mashad SM, van Sinderen D, O'Sullivan GC, et al. Orally administered bifidobacteria as vehicles for delivery of agents to systemic tumors. Mol Ther. 2010;18:1397-407.

## Claims

1. A combination comprising an effective dose of a chemotherapeutic-activating wild-type bacterium and an effective dose of a chemotherapeutic agent that is responsive to the chemotherapeutic activating wild-type bacterium, for use in a method of preventing or treating cancer in a mammal.

2. A combination of Claim 1, for use in a method of preventing or treating cancer in a mammal, wherein the chemotherapeutic-activating wild-type bacterium is a mammalian tumor-resident wild-type bacterium, optionally selected from *E.coli* and *Listeria welshimeri*.

3. A combination of Claim 1 or claim 2, for use in a method of preventing or treating cancer in a mammal, wherein the chemotherapeutic agent is selected from Tegafur, Fludarabine de phosphate, 5-fluorcytosine, 6-mercaptopurine-2'-deoxyriboside, and AQ4N.

4. A combination of any preceding Claim, in which the actives are administered together or separately.

5. A composition comprising a chemotherapeutic-activating wild-type bacterium and a chemotherapeutic agent that is responsive to the chemotherapeutic activating wild-type bacterium.

6. A composition according to Claim 5 wherein the chemotherapeutic-activating wild-type bacterium is a mammalian tumor-resident wild-type bacterium, optionally selected from *E. coli* and *Listeria welshimeri*.

7. A composition according to Claim 5 or 6, wherein the chemotherapeutic agent is selected from Tegafur, Fludarabine de phosphate, 5-fluorcytosine, 6-mercaptopurine-2'-deoxyriboside, and AQ4N.

8. A method for identifying a chemotherapeutic agent that is a responsive to a specific wild-type bacterium comprising the sequential steps of incubating the specific wild-type bacterium with a candidate chemotherapeutic agent, separating the candidate chemotherapeutic agent from the wild-type bacterium, incubating the candidate chemotherapeutic agent with a cancer cell, and determining whether there is an increase in a cytotoxic effect of the candidate chemotherapeutic agent on the cancer cell relative to a reference cytotoxic effect, wherein an increase in cytotoxic effect relative to the reference cytotoxic effect indicates that the candidate chemotherapeutic agent is a wild-type bacterium responsive chemotherapeutic agent.

9. A combination comprising an effective dose of a wild-type bacterium non-responsive chemotherapeutic agent and an effective dose of an anti-bacterial agent effective against the wild-type bacterium, for use in a method for preventing or treating cancer in a mammal.

10. A combination of Claim 9, for use in a method of preventing or treating cancer in a mammal, wherein the chemotherapeutic-activating wild-type bacteria is a mammalian tumor-resident wild-type bacterium, optionally selected from *E.coli* and *Listeria welshimeri*.

11. A combination of Claim 9 or claim 10, for use in a method of preventing or treating cancer in a mammal, wherein the chemotherapeutic agent is selected from Cladribine, Vidarabine, Gemcitabine, Doxorubicin, Daunorubicin, Idarubicin, Etoposide phosphate, Mitoxantrone, B-Lapachone, and Medadione.

12. A combination of any of Claims 9 to 11, in which the actives are administered together or separately.

13. A method of identifying a suitable chemotherapeutic agent for a mammal having a tumour comprising the steps of assaying the tumour for the presence of at least one chemotherapeutic activating wild-type bacterium, wherein when the presence in the tumour of the at least one chemotherapeutic activating wild-type bacterium is confirmed, identifying a chemotherapeutic agent that is responsive to the wild-type bacterium as a suitable chemotherapeutic agent for the mammal.

14. A method of identifying a suitable chemotherapeutic regime for a mammal having a tumour comprising the steps of assaying the tumour for the presence of at least one chemotherapeutic inhibiting wild-type bacterium, wherein when the presence in the tumour of the at least one chemotherapeutic inhibiting wild-type bacterium is confirmed, identifying an anti-bacterial agent effective against the chemotherapeutic inhibiting wild-type bacterium and a chemotherapeutic agent that is non-responsive to the wild-type bacterium as a suitable chemotherapeutic regime for the mammal.

15. A method for identifying a chemotherapeutic agent that is non-responsive to a specific wild-type bacterium comprising the sequential steps of incubating the specific wild-type bacterium with a candidate chemotherapeutic agent for a period of time, separating the candidate chemotherapeutic agent from the wild-type bacterium, incubating the candidate chemotherapeutic agent with a cancer cell, and determining whether there is an decrease in a cytotoxic effect of the candidate chemotherapeutic agent on the cancer cell relative to a reference cytotoxic effect, wherein a decrease in cytotoxic effect relative to the reference cytotoxic effect indicates that the candidate chemotherapeutic agent is a wild-type bacterium non-responsive chemotherapeutic agent.
